# EUROPEAN PATENT APPLICATION

(11) **EP 3 604 268 A1**
(43) Date of publication of application: **05.02.2020**
(21) Application number: 17902856.8
(22) Date of filing: 28.03.2017
(51) Int. Cl.: C07C 209/68, C07C 211/09

(54) **ALIPHATIC AMINE, AND PREPARATION METHOD THEREFOR AND APPLICATIONS THEREOF**

(71) Applicant: Covestro Deutschland AG, 51373 Leverkusen (DE)
(72) Inventor: GUO, Ruijing, Shanghai 201208 (CN); MA, Mingyan, Shanghai 201208 (CN); LIN, Jen-Chieh, 587970 (SG); MEINE, Niklas, 40223 Düsseldorf (DE); JAEGER, Gernot, 50733 Köln (DE); LANGSTEIN, Gerhard, 51515 Kürten (DE); EGGERT, Christoph, 50733 Köln (DE)
(74) Representative: Levpat
(86) International application number: PCT/CN2017/078363
(87) International publication number: WO 2018/176205

(57) **Abstract**

The invention relates to aliphatic amines, methods for preparing the same and use thereof, particularly their use in curing agents. The method for preparing an aliphatic amine comprises reacting a reaction composition comprising an amino acid and an amine compound capable of dissolving the amino acid to obtain the aliphatic amine, wherein the content ratio of the amino acid to the amine compound is 1:10000-1:1, preferably 1:100-1:2, most preferably 1:10-1:2. The method for preparing an aliphatic amine provided by the present invention has a high reaction rate, a high conversion rate of the amino acid, a high purity of the aliphatic product, a long catalyst life, and easy purification of the reaction product.

## Description

### Technical field

The invention relates to an aliphatic amine and its preparation method and use, in particular in the field of curing agents. The invention also relates to a method for the preparation of an aliphatic diisocyanate by using an aliphatic diamine and to the resulting aliphatic diisocyanate.

### Background

Aliphatic amines have attracted much attention as raw materials of polymers from living organisms. Aliphatic amines are usually produced from petroleum feedstocks or amino acids. Due to the increasingly reduced petroleum resources, the industry generally utilizes decarboxylation of amino acids to prepare aliphatic amines.

Decarboxylation of amino acids can be either biological decarboxylation or chemical decarboxylation. Biological decarboxylation is a process of fermentation and decarboxylation. The fermentation process may introduce many impurities including water, which is not conducive to the purification of the final aliphatic amine products. Besides, the process of fermentation and decarboxylation generates large amounts of carbon dioxide, which is not conducive to the environment. Therefore, the industry begins to consider methods of using chemical decarboxylation to prepare aliphatic amines.

G. Laval and B. T. Golding, Synlett, 2003, 542-546, describes the chemical decarboxylation of lysine to prepare pentanediamine in an organic solvent in the presence of an ammonium chloride catalyst.

US 2014/0275569 A1 discloses a method for chemical decarboxylation of amino acids to prepare imines. The reaction solution comprises an amino acid, a solvent and a ketone catalyst. The boiling point of the solvent is lower than that of cyclohexanol. The maximum vapor pressure generated by the solvent does not exceed the ultimate pressure of a vessel. The solvent can be n-propanol or water. The solubility of the amino acid in n-propanol is less than 1 g/100 g. The solubility of the amino acid in water is greater than 1 g/100 g. However, the boiling point is lower than 100°C and thereby this method cannot be used under reaction conditions of normal pressure and a temperature of 150°C.

Chemical letters, 1986, 893-896 describes chemical decarboxylation of amino acids to prepare aliphatic diamines in a solvent of cyclohexanol in the presence of a ketone catalyst. The solubility of amino acids in cyclohexanol is less than 1 g/100 g.

JP 2014152158 A discloses a method for chemical decarboxylation of amino acids to prepare aliphatic diamines. The reaction solution comprises an amino acid, a solvent and an imine catalyst. The solvent can be an alcohol, especially cyclohexanol.

CN 101205160 A discloses a method for chemical decarboxylation of amino acids. The reaction solution comprises an amino acid, a solvent and a carbonyl compound catalyst. The solvent has a boiling point of 150-390°C and a water-entrainment effect and can be 2-ethylhexanol, dibenzyltoluene or isononanol in which the solubility of the amino acid is less than 1 g/100 g. The carbonyl compound catalyst can be a cyclic or acyclic ketone or aldehyde, such as 2-cyclohexen-1-one or isophorone.

The aforementioned reaction systems for chemical decarboxylation of amino acids often use organic solvents such as alcohols, but amino acids cannot be dissolved in these organic solvents, resulting in a very low efficiency of decarboxylation reaction. The aforementioned reaction systems for chemical decarboxylation of amino acids use a ketone compound, ammonium chloride or an imine compound as a homogeneous catalyst which, however, cannot be easily separated from the reaction product. In addition, the homogeneous catalyst not only accelerates the decarboxylation reaction, but also forms an imine intermediate which may in turn deactivate the catalyst.

### Summary of the Invention

The object of the present invention is to provide an aliphatic amine and its preparation method and use, in particular in the field of curing agents.

According to the method for preparing an aliphatic amine of the present invention, the aliphatic amine is obtained by reacting a reaction composition comprising an amino acid and an amine compound capable of dissolving the amino acid, wherein the content ratio of the amino acid to the amine compound is 1:10000-1:1.

According to an embodiment of the present invention, there is provided an aliphatic amine obtained by carrying out the method provided by the present invention, wherein the aliphatic amine has a purity of not less than 95% without purification.

According to an embodiment of the present invention, the aliphatic diisocyanate provided by the present invention is obtained by phosgenation of an aliphatic amine, wherein the aliphatic amine is obtained according to the method provided by the present invention. The aliphatic amine has a purity of not less than 95% without purification. The aliphatic amine is an aliphatic diamine.
The phosgenation is preferably a gas-phase phosgenation.

According to an embodiment of the present invention, the polyamide provided by the present invention is obtained through the reaction of an aliphatic amine and a diacid, wherein the aliphatic amine is obtained according to the method provided by the present invention. The aliphatic amine has a purity of not less than 95% without purification. The aliphatic amine is an aliphatic diamine.

According to an embodiment of the present invention, the polyisocyanate composition provided by the present invention is obtained by modifying an aliphatic diisocyanate, wherein the aliphatic diisocyanate is obtained by phosgenating an aliphatic amine, and the aliphatic amine is obtained according to the method provided by the present invention. The aliphatic amine has a purity of not less than 95% without purification. The aliphatic amine is an aliphatic diamine. The polyisocyanate composition comprises one or more groups selected from isocyanurate, allophanate, biuret, carbamate, and urea groups.

According to an embodiment of the present invention, the polyurethane polymer provided by the present invention is obtained by reacting an aliphatic diisocyanate with an active hydrogen compound, wherein the aliphatic diisocyanate is obtained by phosgenating an aliphatic amine, and the aliphatic amine is obtained according to the method provided by the present invention. The aliphatic amine has a purity of not less than 95% without purification. The aliphatic amine is an aliphatic diamine.

According to an embodiment of the present invention, the polyurethane polymer provided by the present invention is obtained by reacting a polyisocyanate composition with an active hydrogen compound, wherein the polyisocyanate composition is obtained by modifying an aliphatic diisocyanate, and the aliphatic diisocyanate is obtained by phosgenating an aliphatic amine, and the aliphatic amine is obtained according to the method provided by the present invention. The aliphatic amine has a purity of not less than 95% without purification. The aliphatic amine is an aliphatic diamine. The polyisocyanate composition comprises one or more groups selected from isocyanurate, allophanate, biuret, carbamate, and urea groups.

According to an embodiment of the present invention, the method for preparing an aliphatic diisocyanate provided by the present invention comprises the step of phosgenating an aliphatic amine to obtain the aliphatic diisocyanate, wherein the aliphatic amine is obtained according to the method provided by the present invention. The aliphatic amine has a purity of not less than 95% without purification. The aliphatic amine is an aliphatic diamine.
The phosgenation is preferably a gas-phase phosgenation.
The aliphatic diamine obtained according to the method provided by the present invention can be directly phosgenated to obtain an aliphatic diisocyanate without a purification step.

According to an embodiment of the present invention, the method for preparing a polyamide provided by the present invention comprises the step of polymerizing an aliphatic amine and a diacid to obtain the polyamide, wherein the aliphatic amine is obtained according to the method provided by the present invention. The aliphatic amine has a purity of not less than 95% without purification. The aliphatic amine is an aliphatic diamine.
The aliphatic diamine obtained according to the method provided by the present invention can be directly polymerized with a diacid to obtain the polyamide without a purification step. According to an embodiment of the present invention, the method for preparing a polyisocyanate composition provided by the present invention comprises the steps of phosgenating an aliphatic amine to obtain an aliphatic diisocyanate and modifying the aliphatic diisocyanate to obtain the polyisocyanate composition, wherein the aliphatic amine is obtained according to the method provided by the present invention. The aliphatic amine has a purity of not less than 95% without purification. The aliphatic amine is an aliphatic diamine. The polyisocyanate composition comprises one or more groups selected from isocyanurate, allophanate, biuret, carbamate, and urea groups.
The aliphatic diamine obtained according to the method provided by the present invention can be directly phosgenated to obtain the aliphatic diisocyanate without a purification step.

According to an embodiment of the present invention, the method for preparing a polyurethane polymer provided by the present invention comprises the steps of phosgenating an aliphatic amine to obtain an aliphatic diisocyanate and reacting the aliphatic diisocyanate and an active hydrogen compound to obtain the polyurethane polymer, wherein the aliphatic amine is obtained according to the method provided by the present invention. The aliphatic amine has a purity of not less than 95% without purification. The aliphatic amine is an aliphatic diamine.
The aliphatic diamine obtained according to the method provided by the present invention can be directly phosgenated to obtain the aliphatic diisocyanate without a purification step.

According to an embodiment of the present invention, the method for preparing a polyurethane polymer provided by the present invention comprises the steps of phosgenating an aliphatic amine to obtain an aliphatic diisocyanate, modifying the aliphatic diisocyanate to obtain the polyisocyanate composition, and reacting the polyisocyanate composition with an active hydrogen compound to obtain the polyurethane polymer, wherein the aliphatic amine is obtained according to the method provided by the present invention. The aliphatic amine has a purity of not less than 95% without purification. The aliphatic amine is an aliphatic diamine. The polyisocyanate composition comprises one or more groups selected from isocyanurate, allophanate, biuret, carbamate, and urea groups.

The aliphatic diamine obtained according to the method provided by the present invention can be directly phosgenated to obtain the aliphatic diisocyanate without a purification step.

According to an embodiment of the present invention, the present invention provides the use of the aliphatic diamine obtained according to the method provided by the present invention for preparing a curing agent, wherein the aliphatic amine has a purity of not less than 95% without purification.

According to an embodiment of the present invention, the present invention provides a curing agent comprising the aliphatic amine obtained according to the method provided by the present invention, wherein the aliphatic amine has a purity of not less than 95% without purification..

According to an embodiment of the present invention, the present invention provides the use of the aliphatic diamine obtained according to the method provided by the present invention for preparing a polyamide, wherein the aliphatic amine has a purity of not less than 95% without purification, and the aliphatic amine is an aliphatic diamine.

The term "capable of dissolving" means that the solubility of an amino acid in a compound is not less than 1/100 g at 100°C under normal pressure. In the present invention, it is regarded as being insoluble when an amino acid has a solubility of less than 1/100 g in a compound at 100°C under normal pressure.

The present invention uses an amine compound capable of dissolving the amino acid to improve the efficiency of chemical decarboxylation reaction and to increase the amino acid conversion rate. On the other hand, it is preferable to use the same amine compound capable of dissolving the amino acid as the aliphatic amine to be produced, which can simplify the separation step of the aliphatic amine product.

The present invention also preferably uses a solid catalyst, which can not only improve the efficiency of chemical decarboxylation reaction and increase the amino acid conversion rate, but also avoid the formation of an imine intermediate that may deactivate the solid catalyst during the chemical decarboxylation process. Besides, it leads to reduced byproducts and increased purity of the aliphatic amine product. Furthermore, the solid catalyst acts as a heterogeneous catalyst in the chemical decarboxylation reaction and thereby is easily separated from the aliphatic amine product.

The present invention also uses a compound that can provide free hydrogen ions, the free hydrogen ions released by which can facilitate the chemical decarboxylation reaction and increase the amino acid conversion rate.

Therefore, the present invention virtually provides a method for chemically decarboxylating an amino acid to prepare an aliphatic amine with a high chemical decarboxylation reaction efficiency, a high amino acid conversion rate, a high purity of the aliphatic amine product, a long catalyst life, and easy purification of the reaction product.

In addition, the aliphatic diamine provided by the present invention has a purity of not less than 95% without purification, thereby avoiding a purification step before using it as a raw material in the next reaction and thus improving the production efficiency.

### Detailed Description

The present invention provides a method for preparing an aliphatic amine by reacting a reaction composition comprising an amino acid and an amine compound capable of dissolving the amino acid to obtain the aliphatic amine, wherein the content ratio of the amino acid to the amine compound is 1:10000-1:1. The present invention also provides the aliphatic amine prepared by this method and its use, in particular for use in curing agents, in the preparation of aliphatic diisocyanates and in the preparation of polyamides.

### Method for preparing an aliphatic amine

The content ratio of the amino acid to the amine compound is preferably from 1:1000 to 1:1, more preferably from 1:100 to 1:2, and most preferably from 1:10 to 1:2.

### Aliphatic amine

The aliphatic amine can be an aliphatic diamine.
The aliphatic diamine is preferably selected from one or more of pentanediamine, isophoronediamine, 1,10-decanediamine, and diaminocyclohexylmethane, more preferably pentanediamine, most preferably 1,5-pentanediamine.

### Amino acid

The amino acid preferably comprises not less than two amino groups, more preferably comprises two amino groups, and most preferably is lysine.
The amino acid can be present in one or more of the following forms: amino acid salts, amino acid hydrates, amino acid derivatives and treated or untreated amino acid fermentation broths, preferably in the form of amino acid salts.
The amino acid salt can be one or more of the following: amino acid organic acid salts such as formates, acetates, adipates, oxalates, 2-ethylhexanoates, stearates, sebacates, succinates, sulfonates, and amino acid inorganic acid salts such as nitrates, sulfates, hydrochlorides, phosphates, carbonates, bicarbonates. The amino acid salt is preferably an amino acid hydrochloride and/or an amino acid carbonate, most preferably an amino acid hydrochloride.

### Amine compound capable of dissolving the amino acid

The amine compound can be an aliphatic amine or an aromatic amine, preferably an aliphatic amine, more preferably an aliphatic amine having the same or similar structure as the aliphatic amine, and most preferably an aliphatic diamine.
The term "similar" means that both are isomeric to each other or both only differ in the number of carbon atoms.
The aliphatic diamine can be selected from one or more of the following: one or more of pentanediamine, isophoronediamine, 1,10-decanediamine, and diaminocyclohexylmethane, preferably pentanediamine, most preferably 1,5-pentanediamine.
The amine compound can have a boiling point higher than 150°C. The high boiling point of the amine compound allows the chemical decarboxylation reaction to proceed at a relatively high reaction temperature, thereby shortening the reaction time.

### Reaction

In the present invention, reacting a reaction composition comprising an amino acid and an amine compound capable of dissolving the amino acid means that the amino acid undergoes a chemical decarboxylation reaction, wherein the reaction temperature is 100-250°C, most preferably 150-250°C.

### Catalyst

The reaction composition can further comprise a catalyst having a content of not less than 0.1%, preferably not less than 1%, based on the amount of the amino acid as 100 wt%.
The catalyst can be a Bronsted acid catalyst.
The Bronsted acid catalyst is preferably a solid Bronsted acid catalyst.
The solid Bronsted acid catalyst is preferably a molecular sieve catalyst.
The molecular sieve catalyst is preferably a 4A molecular sieve and/or a Y-type molecular sieve.

### Compound that can provide free hydrogen ions

The reaction composition can further comprise a compound that can provide free hydrogen ions, wherein the content of free hydrogen ions is from 0.001 to 10 molar equivalents, based on the molar content of the amine compound capable of dissolving the amino acid as 1 mol.
The content of free hydrogen ions is preferably from 0.001 to 2 molar equivalents, based on the molar content of the amine compound capable of dissolving the amino acid as 1 mol.
The compound that can provide hydrogen ions can be a inorganic acid and/or an organic acid. The inorganic acid can be hydrochloric acid and/or sulfuric acid. The organic acid can be p-toluenesulfonic acid.

### Method for preparing an aliphatic diisocyanate

The aliphatic diamine is preferably 1,5-pentanediamine, and the aliphatic diisocyanate is preferably 1,5-pentamethylene diisocyanate.
The aliphatic diamine can also be present in the form of a salt.
The phosgenation includes a liquid-phase phosgenation, a gas-phase phosgenation, and a solid-phase phosgenation, preferably is a gas-phase phosgenation.

A stabilizer can be added to the prepared 1,5-pentamethylene diisocyanate.
The stabilizer can be an antioxidant, an acidic compound, a compound containing a sulfonamide group, or an organic phosphite. The amount of the stabilizer to be added is not particularly limited, and can be adjusted depending on need and use.
The antioxidant can be a hindered phenolic antioxidant.
The acidic compound can be an organic acidic compound.
The sulfonamide group-containing compound can be an aromatic sulfonamide or an aliphatic sulfonamide.

### Method for preparing a polyamide

The diacid can be an aliphatic diacid. The aliphatic diacid can be prepared by a chemical method or by a biological method.
The aliphatic diacid is preferably selected from one or more of the following: oxalic acid, malonic acid, succinic acid, glutaric acid, adipic acid, pimelic acid, suberic acid, azelaic acid, sebacic acid, undecanedioic acid, dodecanedioic acid, tridecanedioic acid, tetradecanedioic acid, pentadecanedioic acid, hexadecanedioic acid, heptadecanedioic acid and octadecanedioic acid, most preferably dodecanedioic acid, adipic acid or sebacic acid.
The aliphatic diamine is preferably an aliphatic primary diamine, more preferably pentanediamine, hexanediamine or butanediamine, and most preferably 1,5-pentanediamine. The polyamide reaction can be a method known to a person skilled in the art, preferably melt polymerization.
The melt polymerization is preferably performed in the presence of an anti-oxidation catalyst. The anti-oxidation catalyst is preferably phosphoric acid, phosphorous acid, hypophosphorous acid or salts or esters thereof and the like.
The polyamide reaction can also be blended with other ingredients at any stage.
The other ingredients can be one or more of the following: antioxidants, heat stabilizers, weathering agents, anti-blocking agents, lubricants, pigments, dyes, crystal nucleating agents, plasticizers, antistatic agents, flame retardants, fillers and other polymers.
The antioxidant and/or thermal stabilizer can be selected from the group consisting of hindered phenolic compounds, hydroquinone-based compounds, hydroquinone-based compounds, phosphite-based compounds and their substituents, copper halides or iodides.
The weathering agent can be selected from the group consisting of resorcinol-based compounds, salicylate-based compounds, benzotriazole-based compounds, benzophenone-based compounds, or hindered amine-based compounds.
The anti-blocking agent and/or lubricant can be selected from the group consisting of aliphatic alcohols, aliphatic amides, aliphatic bisamides, diureas or polyethylene waxes.
The pigment can be selected from the group consisting of cadmium sulfide, phthalocyanine or carbon black.
The dye can be selected from the group consisting of nigrosin or aniline black.
The crystal nucleating agent can be selected from the group consisting of inorganic fine particles such as talc, boron nitride, or metal oxides, silica, kaolin, clay, or nylon having a high melting point.
The plasticizer can be selected from the group consisting of octyl paraben or N-butyl benzene sulfonamide.
The antistatic agent can be selected from the group consisting of an anionic antistatic agent of alkyl sulfate type, a cationic antistatic agent of quaternary ammonium salt type, a nonionic antistatic agent such as polyoxyethylene sorbitan monostearate, or an amphoteric antistatic agent of betaine type.
The flame retardant can be selected from the group consisting of melamine cyanurate, hydroxides such as magnesium hydroxide, aluminum hydroxide and the like, ammonium polyphosphates, brominated polystyrenes, brominated polyphenyl ethers, brominated polycarbonates, brominated epoxy resins or combinations of these brominated flame retardants with antimony trioxide.
The filler can be selected from the group consisting of granular, acicular or tabular fillers such as glass fiber, carbon fiber, carbon black, black ink, barium sulfate, magnesium sulfate, calcium carbonate, magnesium carbonate, antimonic oxide, titanium dioxide, aluminum oxide, zinc oxide, iron oxide, zinc sulfide, zinc, lead, nickel, aluminum, copper, iron, stainless steel, bentonite, montmorillonoid, manufactured mica and the like.
The other polymers can be selected from the group consisting of polyamides, polyethylenes, polypropylenes, polyesters, polycarbonates, polyphenyl ethers, polyphenyl thioethers, liquid crystal polymers, polysulfones, polyether sulfones, ABS resins, AS resins or polystyrenes. The polyamide can be shaped into a desired shape by means of, for example, injection molding, film molding, melt spinning, blow molding or vacuum forming.

### Polyisocyanate composition

The aliphatic diisocyanate is preferably pentamethylene diisocyanate.
The isocyanurate group-containing polyisocyanate composition can be a trimer of pentamethylene diisocyanate, which can be obtained by trimerization of pentamethylene diisocyanate in the presence of an isocyanuration catalyst.
The allophanate group-containing polyisocyanate composition can be an allophanate modified product of pentamethylene diisocyanate, which can be obtained by the reaction of pentamethylene diisocyanate with a monohydric alcohol and then further reaction in the presence of an allophanatization catalyst.
The biuret group-containing polyisocyanate composition can be a biuret modified product of pentamethylene diisocyanate, which can be obtained by the reaction of pentamethylene diisocyanate, water, a tertiary alcohol and a secondary amine and then further reaction in the presence of a biuretation catalyst.
The carbamate group-containing polyisocyanate composition can be a polyol modified product of pentamethylene diisocyanate, which can be obtained by reacting pentamethylene diisocyanate with a polyol.
The urea group-containing polyisocyanate composition can be a polyamine modified product of pentamethylene diisocyanate, which can be obtained by reacting pentamethylene diisocyanate with water and polyamine.
The aforementioned polyisocyanate composition is preferably a pentamethylene diisocyanate trimer. The method for preparing the pentamethylene diisocyanate trimer can comprise a trimerization reaction of pentamethylene diisocyanate with an alcohol in the presence of a trimerization catalyst, followed by removal of unreacted pentamethylene diisocyanate.
The concentration of isocyanate groups in the polyisocyanate composition is preferably from 10% to 28%.
The mass ratio of the alcohol to pentamethylene diisocyanate is 0.001-0.05, preferably 0.002-0.03.
The alcohol can be a monohydric alcohol, a dihydric alcohol, a trihydric alcohol or a tetrahydric or higher alcohol, preferably a monohydric alcohol and/or a dihydric alcohol, most preferably a monohydric alcohol.
The monohydric alcohol can be a linear monohydric alcohol or a branched monohydric alcohol.
The linear monohydric alcohol can be methanol, ethanol, n-propanol, n-butanol, n-pentanol, n-hexanol, n-heptanol, n-octanol, n-nonanol, n-decanol, n-undecanol, n-dodecanol, n-tridecanol, n-tetradecanol, n-pentadecanol, n-hexadecanol, n-heptadecanol, n-octadecanol, n-nonadecanol, or eicosanol.
The branched monohydric alcohol can be isopropanol, isobutanol, sec-butanol, tert-butanol, isopentanol, isohexanol, isoheptanol, isooctanol, 2-ethylhexan-1-ol, isononanol, isodecanol, 5-ethyl-2-nonanol, trimethylnonanol or 2-hexyldecanol.
The dihydric alcohol can be ethylene glycol, 1,3-propanediol, 1,4-butanediol, 1,5-pentanediol, 1,6-hexanediol, 1,4-dihydroxy-2-butene, diethylene glycol, triethylene glycol, dipropylene glycol, 1,2-propanediol, 1,3-butanediol, 1,2-butanediol, neopentyl glycol, 3-methyl-1,5-pentanediol, 2,2,2-dimethylpentanediol, 3,3-dimethylolheptane, 1,3- or 1,4-cyclohexanedimethanol, 1,3- or 1,4-cyclohexanediol, hydrogenated bisphenol A, or bisphenol A.
The trihydric alcohol can be glycerol or trimethylolpropane.
The tetrahydric or higher alcohol can be tetramethylolmethane, D-sorbitol, xylitol, or D-mannitol.
The alcohol can be used in combination with active hydrogen compounds such as thiols, oximes, lactams, phenols, β-diketones and the like.
The trimerization catalyst is a catalyst having a catalytic effect on trimerization, such as one or more of a hydroxide or an organic weak acid salt of tetraalkylammonium, a hydroxide or an organic weak acid salt of trialkylhydroxyalkylammonium , alkali metal salts of alkyl carboxylic acids, metal chelate compounds, Friedel-Crafts reaction catalysts, organometallic compounds, and aminosilyl-containing compounds.
The mass ratio of the trimerization catalyst to pentamethylene diisocyanate can be from 0.000005:1 to 0.003:1, preferably from 0.00001:1 to 0.001:1, and most preferably from 0.00001:1 to 0.0005:1.
An organic phosphite cocatalyst can also be added in the method for preparing the pentamethylene diisocyanate trimer.
The method for preparing the pentamethylene diisocyanate trimer is preferably carried out in an atmosphere of inert gas, such as nitrogen. The reaction pressure can be normal pressure, the reaction temperature is 30-100°C, preferably 40-80°C, and the reaction time is 0.5-10 hours, preferably 1-5 hours.
The equivalent ratio of the isocyanate groups of pentamethylene diisocyanate to the hydroxyl groups of alcohols is 20 or more, preferably 30 or more, more preferably 40 or more, and most preferably 60 or more, and usually 1000 or less.
The conversion rate of the isocyanate groups of pentamethylene diisocyanate is 5-35 wt%, preferably 5-30 wt%, and most preferably 5-25 wt%. The conversion rate of the isocyanate groups can be measured by high-efficiency GPC, NMR, isocyanate group concentration, refractive index, density, infrared spectrum, and the like.
In the method for preparing the pentamethylene diisocyanate trimer, unreacted pentamethylene diisocyanate is removed by a well known method such as distillation.

### Method for preparing a polyurethane polymer

The polyurethane polymer can be obtained by reacting the above aliphatic diisocyanate and/or the above polyisocyanate composition with an active hydrogen compound.
The active hydrogen compound can be a polyol or a polyamine compound.
The polyol preferably has two or more hydroxyl groups.
The polyol can be a low-molecular-weight polyol having a number-average molecular weight of less than 400 with two or more hydroxyl groups. It can be selected from one or more of the following: ethylene glycol, propylene glycol, 1,3-propanediol, 1,4-butanediol, 1,3-butanediol, 1,2-butanediol, 1,5-pentanediol, 1,6-hexanediol, neopentyl glycol, 3-methyl-1,5-pentanediol, 2,2,2-trimethylpentanediol, 3,3-dimethylolheptane, alkanediol, 1,3- or 1,4-cyclohexanedimethanol and mixtures thereof, 1,3- or 1,4-cyclohexanediol and mixtures thereof, hydrogenated bisphenol A, 1,4-dihydroxy-2-butene, 2,6-dimethyl-1-octene-3, 8-diol, bisphenol A, diethylene glycol, triethylene glycol, dipropylene glycol, glycerol, trimethylolpropane, tetramethylolmethane, diglycerol, xylitol, sorbitol, mannitol, allitol, iditol, dulcitol, altritol, inositol, dipentaerythritol, persitol, and sucrose.
The polyol can also be a high-molecular-weight polyol having a number-average molecular weight of 400 or more with two or more hydroxyl groups. It can be selected from one or more of the following: polyether polyol, polyester polyol, polycarbonate polyol, polyurethane polyol, epoxy polyol, vegetable oil polyol, polyolefin polyol, acrylic polyol and vinyl monomer-modified polyol, preferably polyester polyol and/or acrylic polyol, most preferably polyester polyol.
The polyether polyol can be polypropylene glycol or polytetramethylene ether glycol.
The polyester polyol can be a polycondensate obtained by reacting the low-molecular-weight polyol having a number-average molecular weight of less than 400 with two or more hydroxyl groups with a polyhydric acid under well known conditions.
The polyhydric acid is selected from one or more of the following: oxalic acid, malonic acid, succinic acid, methylsuccinic acid, glutaric acid, adipic acid, 1,1-dimethyl-1,3-dicarboxyl propane, 3-methyl-3-ethylglutaric acid, azelaic acid, sebacic acid, maleic acid, fumaric acid, itaconic acid, phthalic acid, isophthalic acid, terephthalic acid, toluenedicarboxylic acid, naphthalenedicarboxylic acid, hexahydrophthalic acid, dimer acid, hydrogenated dimer acid, hexachloro-endomethylene tetrahydrophthalic acid, anhydrides derived from the aforementioned carboxylic acids such as oxalic anhydride, succinic anhydride, maleic anhydride, phthalic anhydride, 2-alkylsuccinic anhydride, tetrahydrophthalic anhydride, trimellitic anhydride, acyl halides derived from the aforementioned carboxylic acids such as oxalyl dichloride, adipoyl dichloride and sebacoyl dichloride.
The polyester polyol can also be a polyester polyol originating from plants.
The polycarbonate polyol can be an amorphous polycarbonate polyol obtained by copolymerizing a dihydric alcohol such as 1,4-butanediol, 1,5-pentanediol, 3-methyl-1,5-pentanediol, 1,6-hexanediol, with a ring-opening polymer.
The polyurethane polyol can be obtained in the form of polyester polyurethane polyol, polyether polyurethane polyol, polycarbonate polyurethane polyol or polyester polyether polyurethane polyol by reacting the polyester polyol, polyether polyol and/or polycarbonate polyol obtained as described above with an isocyanate. The equivalent ratio of hydroxyl groups of the polyurethane polyol to isocyanate groups of the isocyanate is more than 1. The epoxy polyol can be obtained by reacting the low-molecular-weight polyol having a number-average molecular weight of less than 400 with two or more hydroxyl groups with a polyfunctional halohydrin.
The vegetable oil polyol can be obtained by reacting a castor oil polyol or a castor oil fatty acid with a polypropylene polyol.
The polyolefin polyol can be a polybutadiene polyol or a partially saponified ethylene-vinyl acetate copolymer.
The acrylic polyol can be obtained by copolymerizing a hydroxy-containing acrylate with a copolymerizable vinyl monomer capable of copolymerizing with the hydroxy-containing acrylate. The acrylic polyol is preferably a siloxane polyol or a fluorine-containing polyol. The polyamine compound preferably has two or more amino groups.
The polyamine compound is selected from one or more of the following: aromatic polyamines, araliphatic polyamines, alicyclic polyamines, aliphatic polyamines, amino alcohols, alkoxysilyl compounds having primary amino groups or primary and secondary amino groups, and polyoxyethylene-containing polyamines.
The aromatic polyamine can be 4,4'-diphenylmethane diamine and/or toluene diamine.
The araliphatic polyamine can be 1,3- and/or 1,4-xylene diamine.
The alicyclic polyamine can be selected from one or more of the following: 3-aminomethyl-3,5,5-trimethylcyclohexylamine, 4,4'-dicyclohexylmethane diamine, 2,5(2,6)-bis(aminomethyl)bicyclo[2.2.1]heptane, 1,4-cyclohexane diamine, 1-amino-3-aminomethyl-3,5,5-trimethylcyclohexane, bis-(4-aminocyclohexyl)methane, diaminocyclohexane, 3,9-bis(3-aminopropyl)-2,4,8,10-tetraoxaspiro[5.5]-undecane and 1,3- and 1,4-bis(aminomethyl)cyclohexane.
The aliphatic polyamine can be ethylenediamine, propanediamine, 1,3-propanediamine, 1,4-butanediamine, 1,5-pentanediamine, 1,6-hexanediamine, hydrazine, hydrazine hydrate, diethylenetriamine, triethylenetetramine, tetraethylenepentamine, 1,2-diaminoethane, 1,2-diaminopropane, or 1,3-diaminopentane.
The amino alcohol can be N-(2-aminoethyl)ethanolamine.
The alkoxysilyl compound having primary amino groups or primary and secondary amino groups can be a alkoxysilyl-containing monoamine such as γ-aminopropyltriethoxysilane, N-phenyl-γ-aminopropyltrimethoxysilane and the like; N-β (aminoethyl) γ-aminopropyltrimethoxysilane, or N-β (aminoethyl) γ-aminopropylmethyldimethoxysilane. The polyoxyethylene group-containing polyamine can bea polyoxyalkylene ether diamine, such as polyoxyethylene ether diamine.
The polyamine compound can be used alone or in combination of two or more.
A well known additive can be added to the reaction for preparing the polyurethane polymer. The additive is selected from one or more of the following: plasticizers, anti-blocking agents, heat stabilizers, light stabilizers, antioxidants, mold release agents, catalysts, pigments, dyes, lubricants, fillers and antihydrolysis agents. The additive can be added at the time of synthesizing each reactant, or can be added at the time of mixing or dissolving each reactant, or can be added after synthesis.
The polyurethane polymer can be produced by a polymerization method such as bulk polymerization or solution polymerization.
The bulk polymerization can be carried out by stirring an aliphatic diisocyanate and/or polyisocyanate composition under a nitrogen stream while adding an active hydrogen compound thereto to react at 50-250°C, preferably 50-200°C for 0.5-15 hours.
The solution polymerization can be carried out by adding an aliphatic diisocyanate and/or polyisocyanate composition and an active hydrogen compound to an organic solvent to react at 50-120°C, preferably 50-100°C for 0.5-15 hours.
The method for preparing the polyurethane polymer can remove unreacted aliphatic diisocyanate and/or polyisocyanate composition by a well known method such as distillation or extraction.
In the bulk polymerization or solution polymerization, the equivalent ratio of isocyanate groups in the aliphatic diisocyanate and/or polyisocyanate composition to active hydrogen groups and/or hydroxyl groups and/or amino groups in the active hydrogen compound can be 0.75-1.3, preferably 0.9-1.1.

### Examples

The conversion rate of an amino acid was measured by the SBA-50B Biosensing Analyzer of the Institute of Biology, Shandong Academy of Sciences.

The purity of the reaction product aliphatic amine was measured with a gas chromatograph. The procedure comprises the steps of preparing a 5% (mass fraction) aliphatic amine solution with methanol as an organic phase, setting the vaporization temperature of the aliphatic amine solution at 250°C, and injecting 0.2 µL of the sample. The initial temperature of the gas chromatography column is 120°C; after 2 minutes at the constant temperature, the temperature was raised to 200°C at a rate of 10°C/min; after holding for 20 minutes, the aliphatic amine and lysine were separated on a non-polar column with 100% polydimethylsiloxane as a stationary phase. Finally, the purity of the aliphatic amine was calculated with an area percentage method.

All percentages in the present invention are by weight unless otherwise specified.

The analytical measurements in the present invention were performed at 23°C unless otherwise specified.

**Raw materials and reagents**

| | |
|---|---|
| Lysine | Chemical name L-lysine, purchased from Ningbo Create-Bio Engineering Co., Ltd., used as the amino acid. |
| 1,5-Pentanediamine | Purchased from Cathay Industrial Biotech, used as the amine compound capable of dissolving an amino acid. The solubility of lysine in 1,5-pentanediamine is 33 g/100 g, measured at 100°C under normal pressure. |
| Isophoronediamine | Purchased from TCI (Shanghai) Development Co., Ltd., used as the amine compound capable of dissolving an amino acid. The solubility of lysine in isophoronediamine is 5.2 g/100 g, measured at 100°C under normal pressure. |
| 1,6-Hexanediamine | Purchased from Shanghai Macklin Biochemical Co., Ltd., used as the amine compound capable of dissolving an amino acid. The solubility of lysine in 1,6-hexanediamine is 4.6 g/100 g, measured at 100°C under normal pressure. |
| Ethylene glycol | Purchased from Sinopharm Chemical Reagent Co., Ltd. The solubility of lysine in ethylene glycol is 20 g/100 g, measured at 100°C under normal pressure. |
| Cyclohexanol | Purchased from Shanghai Lingfeng Chemical Reagent Co., Ltd. The solubility of lysine in cyclohexanol is 0.01 g/100 g, measured at 100°C under normal pressure. |
| Zeolite 4A | Molecular sieve catalyst, purchased from Shanghai Shentan Environmental New Material Co., Ltd. and used as the catalyst. |
| Zeolite Y | Molecular sieve catalyst, purchased from Shanghai Shentan Environmental New Material Co., Ltd. and used as the catalyst. |
| Amberlyst-15 | Solid Bronsted acid catalyst, purchased from Shanghai Aladdin Bio-Chem Technology Co., Ltd., used as the catalyst. |
| L-carvone | Ketone catalyst, purchased from TCI (Shanghai) Development Co., Ltd. |
| Microcycloalkenone | Ketone catalyst, purchased from TCI (Shanghai) Development Co., Ltd. |
| Hydrochloric acid | Purchased from Shanghai Gaoxin Chemical Glass Instruments Co., Ltd., with a concentration of 37% and used as the compound that can provide free hydrogen ions. |

### Examples 1-5, Examples 7-10

A container was evacuated and then filled with nitrogen. Repeat these three times. Under an atmosphere of nitrogen, an amine compound capable of dissolving an amino acid was added to the container, and the mixture was heated to the reaction temperature under condensation and reflux. Subsequently, an amino acid, a catalyst, and a compound that can provide free hydrogen ions were added to the container under an atmosphere of nitrogen to obtain a reaction composition, and magnetic stirring was started until the reaction composition was completely dissolved, followed by reaction at a reaction temperature under normal pressure to obtain a reaction product.

### Example 6

A 250-mL high-pressure reactor (EasyChem E250 micro high-pressure reactor, Beijing Century Senlong Experimental Apparatus Co., Ltd.) was evacuated and then filled with nitrogen. Repeat these three times. Under an atmosphere of nitrogen, 1,5-pentanediamine, lysine, and Y-type zeolite catalyst were added to the reactor. The high-pressure reactor was then closed, stirring was started, and nitrogen supply was stopped. After reaction at 220°C under 1 bar for 10 minutes, the temperature was reduced to room temperature to obtain a reaction product. The conversion rate of lysine was 94%, and the purity of 1,5-pentanediamine in the organic phase of the reaction product was 96.82%.

### Comparative Example 1-2

The procedure is the same as that of Examples 1-5 and Examples 7-10 except that the amine compound capable of dissolving the amino acid is replaced with ethylene glycol or cyclohexanol.

The conversion rates of the amino acids and the purity of the reaction products of Examples 1-10 and Comparative Examples 1-2 are measured and listed in Table 1. The reaction components and reaction conditions are also listed in Table 1.

**Table 1: Reaction Components, Reaction Conditions, Conversion Rates of Amino Acids, and Purity of Aliphatic Amine Products of Inventive and Comparative Examples**

| | Amino acid (g) | Amine compound (g) | | | Ethylene glycol | Cyclohexanol | Catalyst (g) | | | | | Compound that can provide free hydrogen ions (molar equivalents) | Reaction temperature (°C) | Reaction time (h) | Amino acid conversion rate (%) | Purity of aliphatic amine product (1,5-pentanediamine) (%) |
|---|---|---|---|---|---|---|---|---|---|---|---|---|---|---|---|---|
| | Lysine | 1,5-Pentanediamine | Isophoronediamine | 1,6-Hexanediamine | | | Zeolite 4A | Zeolite Y | Amberlyst -15 | L-carvone | Microcycloalkenone | hydrochloric acid | | | | |
| Example 1 | 4.9 | 10 | | | | | | | | | | | 180 | 1.5 | 85 | 95.65 |
| Example 2 | 4.9 | 10 | | | | | 0.15 | | | | | | 180 | 1.5 | 89 | 96.52 |
| Example 3 | 4.9 | 10 | | | | | 0.15 | | | | | 0.0014 | 180 | 1.5 | 91 | 99.12 |
| Example 4 | 1 | 10 | | | | | | 2 | | | | | 150 | 1.5 | 88 | 98.42 |
| Example 5 | 1 | 10 | | | | | | | 0.1 | | | | 180 | 1.5 | 84 | 97.6 |
| Example 6 | 30 | 60 | | | | | | 1 | | | | | 220 | 0.17 | 94 | 96.82 |
| Example 7 | 1 | 10 | | | | | | | | | | | 200 | 1 | 90 | |
| Example 8 | 1 | | 10 | | | | | | | | | | 200 | 1 | 94 | |
| Example 9 | 1 | | | 10 | | | | | | | | | 200 | 1 | 94 | |
| Example 10 | 4.9 | 10 | | | | | | | | 0.3 | | | 150 | 3 | 88 | |
| Comp. Ex. 1 | 4.9 | | | | 10 | | 0.15 | | | | | | 160 | 3 | 77 | 84.4 |
| Comp. Ex. 2 | 4.9 | | | | | 10 | | | | | 0.15 | | 160 | 4.5 | <1 | |

When comparing Examples 1-3, 10 and Comparative Example 1, Comparative Example 1 uses ethylene glycol solvent, and the conversion rate of the amino acid and the purity of the aliphatic amine product are significantly lower than those in the chemical decarboxylation reaction using an amine compound.

When comparing Examples 1-2 and Comparative Example 2, Comparative Example 2 uses cyclohexanol solvent that cannot dissolve lysine, and the conversion rate of the amino acid and the purity of the aliphatic amine product are significantly lower than those in the chemical decarboxylation reaction using an amine compound capable of dissolving lysine.

When comparing Examples 1-2 and Comparative Example 2, Comparative Example 2 uses a ketone catalyst, i.e. a homogeneous catalyst, and the conversion rate of the amino acid and the purity of the aliphatic amine product are significantly lower than those in the chemical decarboxylation reaction using a heterogeneous catalyst.

When comparing Examples 2-3 and Example 10, in case of the chemical decarboxylation reaction using a molecular sieve catalyst, the conversion rate of the amino acid is higher than that in the chemical decarboxylation reaction using a homogeneous ketone catalyst.

When comparing Example 2 and Example 3, in case of the chemical decarboxylation reaction using a compound that can provide free hydrogen ions, the conversion rate of the amino acid and the purity of the aliphatic amine product are increased.

It will be apparent to those skilled in the art that the present invention is not limited to the specific details described above, and that the present invention can be embodied in other specific forms without departing from the spirit or essential characteristics of the present invention. The described examples are therefore to be considered in all respects as illustrative and not restrictive so that the scope of the invention is indicated by the appended claims rather than by the foregoing description; and any modifications therefore should be regarded as belonging to the present invention, as long as they fall within the meaning and scope of the equivalents of the claims.

## Claims

1. A method for preparing an aliphatic amine, **characterized by** reacting a reaction composition comprising an amino acid and an amine compound capable of dissolving the amino acid to obtain the aliphatic amine, wherein the content ratio of the amino acid to the amine compound is from 1:10000 to 1:1, preferably from 1:1000 to 1:1, more preferably from 1:100 to 1:2, and most preferably from 1:10 to 1:2.

2. The method according to claim 1, **characterized in that** the aliphatic amine is an aliphatic diamine, preferably pentanediamine.

3. The method according to claim 1, **characterized in that** the amino acid comprises not less than two amino groups, preferably two amino groups, and most preferably, it is lysine.

4. The method according to claim 1, **characterized in that** the amine compound capable of dissolving the amino acid is an aliphatic amine having the same or similar structure as the aliphatic amine, preferably an aliphatic diamine, most preferably pentanediamine.

5. The method according to claim 1, **characterized in that** the amine compound has a boiling point higher than 150°C.

6. The method according to claim 1, **characterized in that** the temperature of the reaction is from 50 to 350°C, preferably from 100 to 250°C, most preferably from 150 to 250°C.

7. The method according to claims 1-6, **characterized in that** the reaction composition further comprises a catalyst, wherein the content of the catalyst is from 0.01% to 100%, preferably from 0.1% to 100%, based on the amount of the amino acid as 100 wt%.

8. The method according to claim 7, **characterized in that** the catalyst is a Bronsted acid catalyst, preferably a solid Bronsted acid catalyst, most preferably a molecular sieve catalyst.

9. The method according to claims 1-6, **characterized in that** the reaction composition further comprises a compound that can provide free hydrogen ions, wherein the content of the free hydrogen ions is from 0.01 to 10 molar equivalents, preferably from 0.1 to 2 molar equivalents, based on the molar content of the amine compound as 1 mol.

10. The method according to claim 9, **characterized in that** the compound that can provide hydrogen ions can be an inorganic acid and/or an organic acid.

11. An aliphatic amine obtained by carrying out the method of any one of claims 1 to 10, wherein the aliphatic amine has a purity of not less than 95% without purification.

12. An aliphatic diisocyanate obtained by phosgenating an aliphatic amine obtained according to the method of any one of claims 1 to 10, wherein the aliphatic amine has a purity of not less than 95% without purification, and the aliphatic amine is an aliphatic diamine.

13. A method for preparaing an aliphatic diisocyanate, **characterized in that** phosgenation of an aliphatic amine leads to the aliphatic diisocyanate, wherein the aliphatic amine is obtained according to the method of any one of claims 1 to 10, wherein the aliphatic amine has a purity of not less than 95% without purification, and the aliphatic amine is an aliphatic diamine.

14. The method according to claim 13, **characterized in that** without purification, the aliphatic diamine is directly phosgenated to obtain the aliphatic diisocyanate.

15. Use of the aliphatic amine obtained according to the method of any one of claims 1 to 10 for the preparation of a curing agent, wherein the aliphatic amine has a purity of not less than 95% without purification.

16. A curing agent comprising the aliphatic amine obtained according to the method of any one of claims 1 to 10, wherein the aliphatic amine has a purity of not less than 95% without purification.
